# EUROPEAN PATENT SPECIFICATION

(11) **EP 0 309 559 B1**
(45) Date of publication and mention of the grant of the patent: **21.08.1996**
(21) Application number: 88903743.8
(22) Date of filing: 14.04.1988
(51) Int. Cl.: C12N 15/00, C07G 17/00, C07H 21/04, C12P 19/34

(54) **METHOD FOR PRODUCING TRANSGENIC PIGS**
METHODE ZUR HERSTELLUNG TRANSGENETISCHER SCHWEINE
METHODE POUR LA PREPARATION DES PORCS TRANSGENIQUES

(30) Priority: 14.04.1987 AU 1427/87; 10.11.1987 AU 5326/87
(43) Date of publication of application: 05.04.1989
(73) Proprietor: BresaGen Limited, Adelaide South Australia (AU)
(72) Inventor: SEAMARK, Robert, Frederick, Beaulah Park, S.A. 5067 (AU); WELLS, Julian, Richard, Este, College Park, S.A. 5069 (AU)
(74) Representative: Crisp, David Norman
(86) International application number: AU8800109
(87) International publication number: WO8808026

(56) References cited:
- EP-A- 0 177 343
- WO-A-82/04443
- AU-A- 1 933 183
- AU-A- 2 092 983
- HAMMER, R.E. et. al. "Production of Transgenic Rabbits, Sheep and Pigs by Microinjection". Nature, Volume 315, issued 20 June 1985, (MacMillan Journals Ltd, London, England), see pages 680 to 683.
- PALMITER, R.D. et. al. "Metallothionein-Human GH Fusion Genes Stimulate Growth of Mice". Science, Volume 222, issued 18 November 1983, (American Association for the Advancement of Science, Washington D.C., USA,) see pages 809 to 814.
- PALMITER, R.D. et. al. "Dramatic Growth of Mice that Develop from eggs Microinjected with Metallothionein-Growth Hormone Fusion Genes". Nature, Volume 300, issued 16 December 1982, (MacMillan Journals Ltd, London, England, see pages 611 to 615.
- VAN DER PUTTEN, H. et. al. "Developmental Fate of a Human Insulin Gene in a Transgenic Mouse". Molecular and General Genetics, Volume 198, issued 1984, (Springer-Verlag, Heidelberg, West Germany), see pages 128 to 138.
- BURKI, K. and A. ULLRICH. "Transplantation of the Human Insulin Gene into Fertilized Mouse Eggs". The EMBO Journal, Volume 1, No. 1 issued 1982, (IRL Press Limited, Oxford, England), see pages 127 to 131.
- PURSEL, V.G. et. al. "Development of 1-Cell and 2-Cell Pig ova after Microinjection of Genes". Journal of Animal Science, Volume 65, Supplement 1, issued 1987, (American Society of Animal Science, Champaign, USA), see page 402 (Abstract).
- VIZE, P.D. and J.R.E. WELLS "Isolation and Characterization of the Porcine Growth Hormone Gene". Gene, Volume 55, issued 1987 (Elsevier Scientific publishing Company, Amsterdam, Netherlands), see pages 339 to 344.

## Description

This invention relates to animal husbandry and in particular to methods of creating transgenic pigs having increased growth.

In traditional breeding processes it has been possible to achieve animals with particular desired characteristics. However, many unwanted, as well as the desired characteristics are often obtained in such processes.

Recently there has been major developments relating to the introduction of exogenous genes into the gene line of animals. In general, this is achieved by the use of both R/DNA technology (which includes isolation and
characterisation of a gene) and one-cell embryo techniques, including egg collection and re-implantation. In summary the overall method involves the following steps:
- isolation of a pure gene sample (a fragment of DNA;
- collection of a recently fertilised egg;
- injection of a tiny portion (e.g. 10⁻⁹ of one cubic centimetre) of the gene solution into the male nucleus (from the sperm) before it fuses with the female nucleus to form the one-cell embryo; and
- implanting the injected egg into a suitably prepared surrogate mother.

After the offspring is born, a small portion of tissue (usually the tail) is taken and DNA is prepared therefrom which can be assessed to establish whether the gene injected at the one-cell stage has been stably incorporated into the animal's chromosomes. If so, it is transgenic.

The process described above has been successfully used to accelerate growth and increase size in mice by the introduction of a gene construct incorporating exogenous genes, such as human growth hormone. However, the process has not been successful in attempts to enhance desired characteristics in farm animals.

We have now found it possible to overcome, or at least alleviate, one or more of the difficulties and/or deficiencies related to the prior art processes.

According to one aspect of the present invention there is provided a method for preparing transgenic pigs said method comprising the steps of:
(a) obtaining a recently fertilized pig ovum;
(b) isolating a first DNA sequence encoding a promotor region which promotes expression of porcine growth hormone in a pig;
(c) inserting the first DNA sequence into a plasmid cloning vector;
(d) isolating a second DNA sequence encoding a protein having porcine growth hormone activity in a transgenic pig said second DNA sequence modified at the 3' end to remove repeated sequences;
(e) inserting the second DNA sequence into the plasmid cloning vector at a suitable site;
(f) introducing the insert from plasmid cloning vector into the male pronucleus of said fertilized pig ovum prior to fusion with the female nucleus to form a single cell embryo;
(g) subsequently implanting the ovum into a female pig.

The fertilised ovum may be obtained by any known method.

The gene sample may be isolated by any known method.

A plasmid expression vector which may be used for use in the method of the present invention includes a first cloned sequence of DNA encoding a non-porcine promoter region and a second cloned sequence of DNA encoding porcine growth hormone (PGH) activity.

A plasmid expression vector so formed may include a complete copy of the porcine growth hormone in the coding region.

Any suitable plasmid expression vector or cloning vector may be used. For example the plasmid cloning vector pUC19 (Nonander et al, 1983 Gene 26: 101-106) has been found to be suitable.

The first cloned sequence of DNA may encode a human promoter region. The human promoter region may be the human metallotionein promoter (hMTIIA). In a preferred form, a modified metallothionein promoter plasmid may be used.

The first cloned sequence of DNA may form an Eco RI - Hind III insertion in the plasmid cloning vector. This insertion may be approximately 810 - 823bp in length. In a preferred embodiment it is 823bp in length. The first cloned sequence of DNA may also contain elements of the human metallothioneine promoter.

The DNA sequence coding for porcine growth hormone activity may be isolated from a cDNA library formed from messenger or polyadenylated RNA. For example this may be isolated from porcine pituitary tissue.

The second cloned sequence of DNA may form an Eco RI - Eco RI insertion in the plasmid expression vector. This insertion may be approximately 800 bp in length. The plasmid is a small, high copy number expression vector. The plasmid expansion vector may be plasmid pUC19 or gKT52.

The plasmid for use in the method of the present invention may further include a third cloned sequence of DNA. The third cloned sequence may include the 3' end of the porcine growth hormone gene. The third cloned fragment may form a Sma : Bam/Sma insertion in the second cloned fragment of DNA. This insertion may be approximately 1000 bp in length.

The 3' end fragment may also be modified. In one embodiment the certain regions identified as repeated sequences are deleted. For example the repeats may be deleted to leave approximately 200 base-pairs of 3' fragment as opposed to approximately 1000bp in the original sequence. The modifying of the 3' end of the porcine growth hormone DNA sample to remove repeated sequences is an additional contributing factor to stabilise chromosomal rearrangements of transgenes in the pig genome. This stabilisation may also be achieved by including introns in the porcine gene coding region.

It has been found surprisingly that the inclusion of the 3' end fragment to the coding region may also provide added stability to the read-out sequence (the messenger RNA or mRNA from the gene.

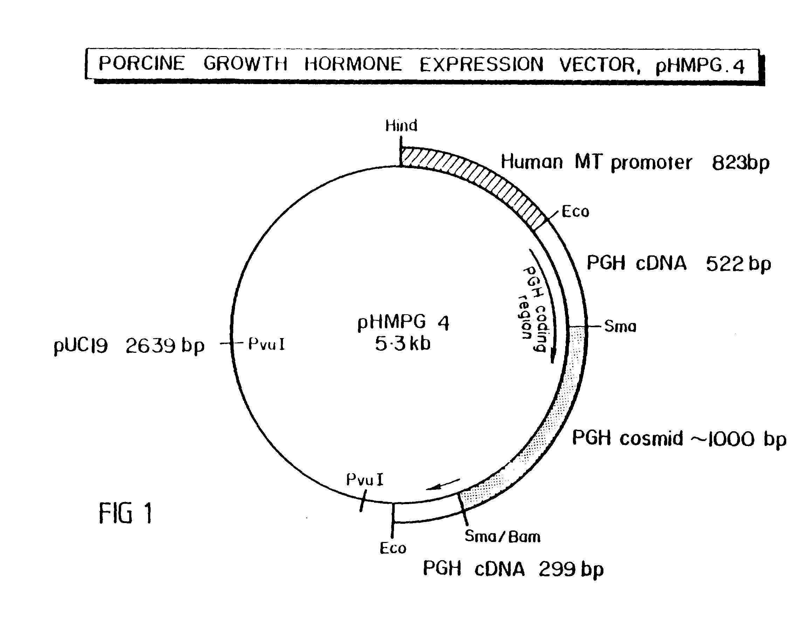

A particular plasmid of the type described above which may be used in the method of the present invention is that designated pH MPG.4, a sample of which is maintained in the culture collection of the University of Adelaide, Australia. However, the plasmid pHMPG.4 is preferably used. A diagram of the pHMPG.4 construct is provided in Figure 1.

A plasmid expression vector such as those described above may be prepared by
- providing a suitable plasmid cloning vector, a first fragment of DNA containing a non-porcine promoter, and a second fragment of DNA coding for porcine growth hormone;
- inserting the first fragment of DNA into the plasmid cloning vector at a suitable site; and
- inserting the second fragment of DNA into the plasmid cloning vector at a suitable site.

The method according to this aspect of the present invention may further include:
- providing a third cloned fragment of DNA including the 3' end of the porcine growth hormone gene from the chromosomal copy of the porcine growth hormone gene.

This further step may be achieved by cleaving the restricted plasmid expression vector at a restriction site for the porcine growth hormone DNA portion and cloning the third fragment of DNA into the second fragment at a suitable site.

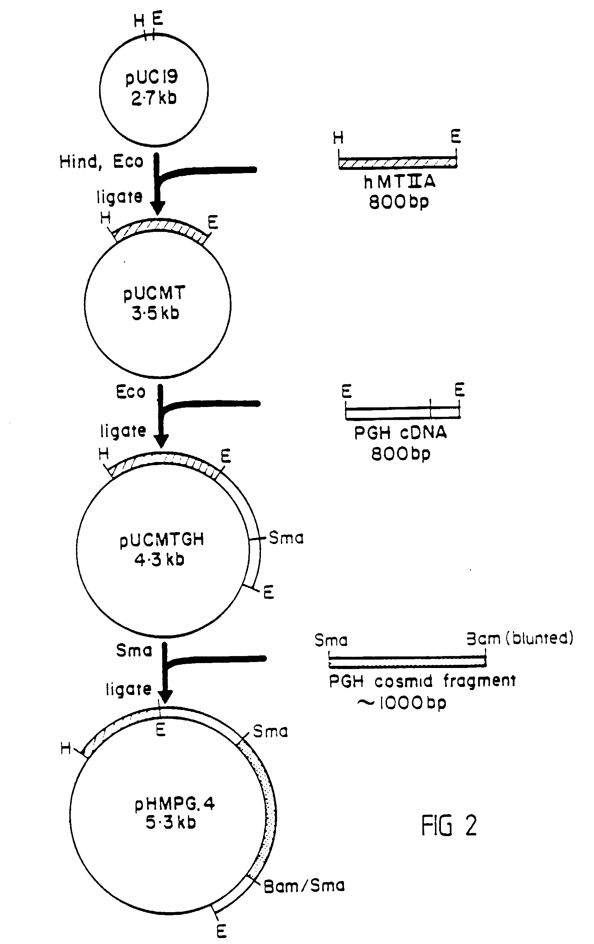

These steps are summarised in Figure 2.

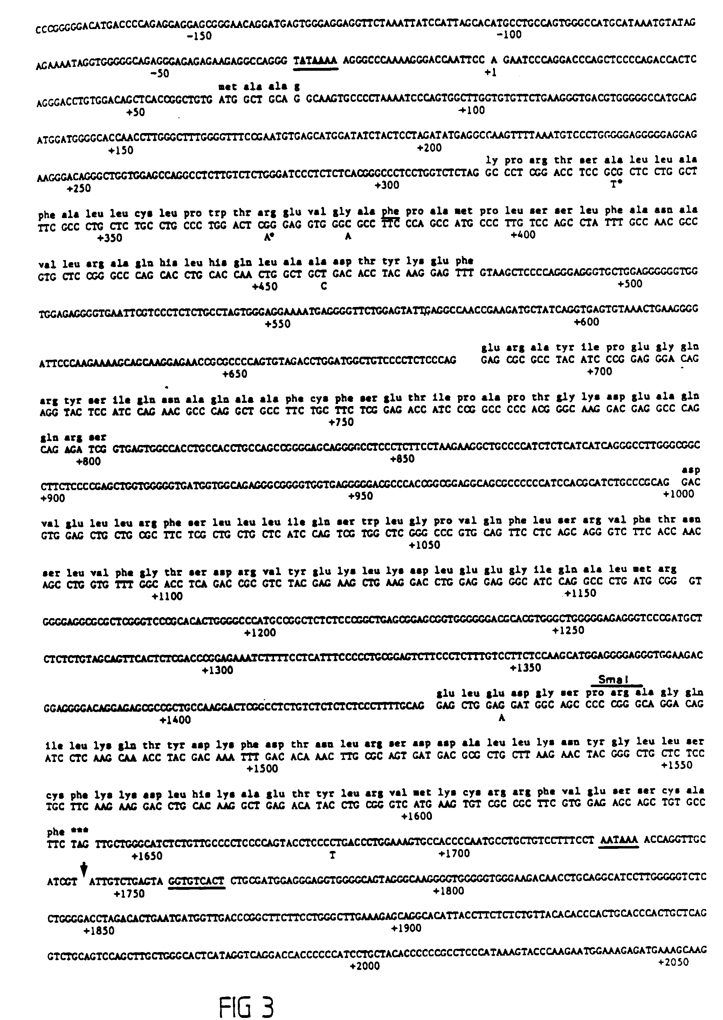

It will be understood that the method of preparing a plasmid so described may include the preliminary step of characterising the coding region and when relevant the 3' non-coding region of porcine growth hormone. This characterisation of porcine growth hormone is described below. The nucleotide sequence of the PGH genomic gene is illustrated in Figure 3.

The plasmid or gene sample, for example the porcine growth sample Hind III/PvuI segment derived from the plasmid shown in Figure 1, may be introduced into the male pronucleus of an ovum of a porcine animal by any suitable method. The gene sample may be injected into the male pronucleus of the ovum.

A major problem encountered with the injection of the gene construct into the male pronucleus is visualisation of the pronuclei or nuclei in the ova. Nuclear structures of such animals as rabbits may be readily seen. However, pronuclei and nuclei in such animals as pigs are difficult to locate.

Pig ova are opaque and no nuclear structures can be seen even with interference contrast microscopy. However, the pronuclei or nuclei of pig ova may be visible if natural pig ova is centrifuged at 15000 g for 3 min.

The injection of the pronucleus may be carried out under magnification and use of standard microinjection apparatus. The ova may be held by a blunt holding pipette and the zona pellucida, plasma membrance and pronuclear envelope may be penetrated by an injection pipette. The blunt holding pipette may have a diameter of approximately 50 µm. The injection pipette may have a diameter of approximately 1.5 µm.

The amount of gene construct injected will necessarily be dependent upon the size of the pronucleus and the size of the injection pipette. For example, a few hundred copies of a 2.6 kilobase (kb) linear fragment containing the desired gene characteristic hormone may be injected.

The egg may be subsequently implanted by any suitable method into any suitably prepared surrogate mother. This may be achieved by any known method.

To further illustrate the invention the following non-limiting example is provided:

### EXAMPLE 1

### PGH cDNA sequences

A porcine pituitary cDNA library of approximately 4000 individual recombinants constructed using the plasmid vector pUC19 (Norrander et al., Gene, 26, 101-106 1983) allowed the isolation of PGH cDNA clones using a synthetic DNA probe. One of the cDNA clones isolated, pPG.3, which was found to contain an insert of the expected full length, was completely sequenced.

The open reading frame of the pPG.3 cDNA insert was found to code for a 216 amino acid pre-hormone, identical in amino acid sequence to the partial length PGH cDNA clone of Seeburg et al. DNA, 2, 37-45 (1983). This clone provided for the first time the complete sequence of the region encoding the PGH signal sequence and 41 basis of the 5' untranslated region.

The nucleotide sequence within the coding region is very highly conserved, with only a single base difference from the sequence of Seeburg et al (1983).

### Figure 3

### Nucleotide sequence of the PGH genomic gene

The complete 2231 bp sequence is illustrated. The open reading frame which pre-PGH is indicated, as are bases which differ in sequence from the previously studied PGH cDNA sequence,pPG.3. Base changes which result in animo acid substitutions are marked with an asterisk. The single base in the 3' untranslated region which differs from pPG.3 is also indicated below the sequence. The location of the cap site (+1) was inferred from the position of the rat and human GH gene cap sites (Page 35 al., 1981; DeNoto et al., 1981).

Putative promoter and polyadenylation sequence, such as the TATA, AATAAA and GT-rich sequences are underlined, and the position at which the poly A tail is added is indicated with an arrow. The variant GC donor splice site is located in the first intron, around base +72.

### Southern analysis using a PGH cDNA hybridization probe

When the cDNA insert of pPG.3 was used to probe Southern blots of porcine genomic DNA digested separately with three different restriction enzymes, a single strongly hybridizing band was detected in each track. Enzymes BamHI and EcoRI also produced a second, fainter band, indicating that if the gene was in fact present as a single copy, the genomic gene must contain internal sites for both of these enzymes, somewhere near the end of the region homologous to the cDNA (as was later discovered to be correct). The third enzyme used, Hind III, produced only a single clear band. Taken together these data indicated that only a single copy of the GH gene exists in the porcine genome (per hapoid chromosome complement), a situation analogous to the bovine and rat genomes, but very different to that of humans.

### Isolation and analysis of the genomic PGH gene

The PGH cDNA clone pPG.3 was used to screen a porcine cosmid library and isolate a clone containing PGH gene sequences (2.4). Southern analysis of the cosmid clone identified major BamHI and EcoRI hybridizing bands where were equal in size to those detected in genomic Southerns utilizing the same hybridization probe. Nucleotide sequencing of the gene contained within the cosmid revealed that the entire coding region was present (648 bp), along with 178 bp of the promoter region, 61 bp of 5' untranslated sequence, four introns of 242, 210, 197 and 278 bp respectively, and 414 bp of 3' non-coding sequence (Figure 3). The gene contains four base alterations within the coding region relative to the previously sequenced PGH cDNA clone, pPG.3. The base substitutions, which are illustrated in Figure 3, result in the alteration of two amino acid residues within the signal peptide. The remaining two differences are silent substitutions. There is also one base change in the 3' untranslated region relative to the pPG.3 cDNA sequence (Figure 3).

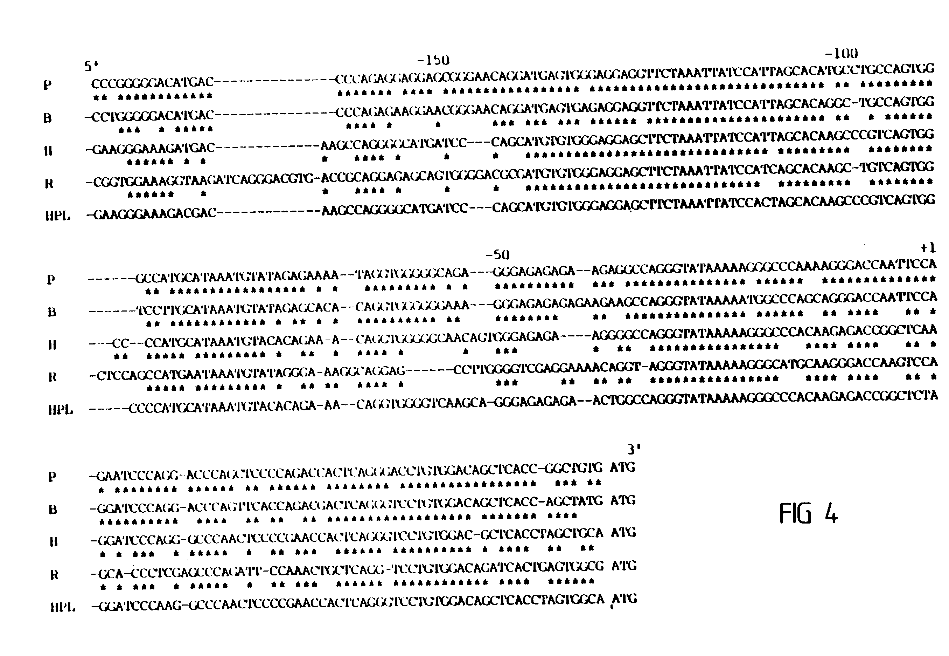

The sequence comparison illustrated in Figure 4 indicates that the promoters of each of the studied sequences share a high degree of homology. Figure 3 indicates that the 5' untranslated regions of the GH and HPL genes are also conserved to a surprising degree.

Analysis of the 3' sequences of the PGH gene allowed the identification of sequences which have been shown to be important in the polyadenylation process.

The comparison of the 3' sequences of the PGH gene to all of the available GH and HPL squences has revealed that these are also conserved to a surprising extent, between both GH and HPL genes.

### Figure 4

### Comparison of GH and HPL gene promoter and 5' untranslated sequences

Porcine (P), bovine (B), human (H) and rat (R) GH genes and the human placental lactogen (HPL) gene were aligned to determine the extent of sequence homology (2.5.3). Asterisks indicate homology between adjacent sequences. The PGH sequence is numbered with respect to the distance from the cap site (+1).

This example describes the cloning of the porcine GH cDNA insert contained in pPG.3 into a bacterial expression vector.

### Expression of PGH in E.coli

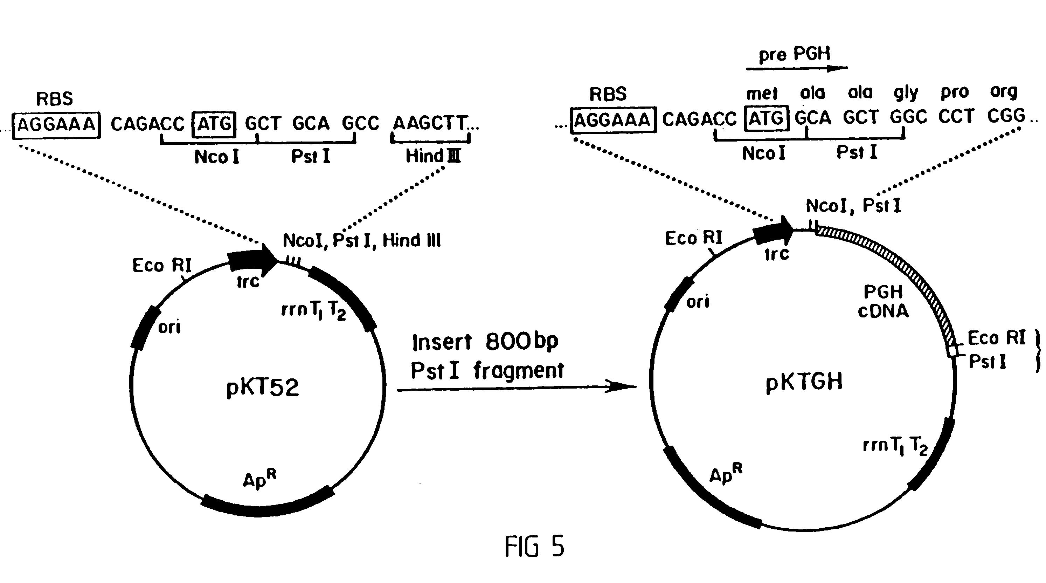

The expression vector chosen for the production of PGH in E.coli cells was pKT52. This plasmid, kindly provided by J. Shine (California Biotechnology), is a small, high copy number expression vector which contains a powerful, regulatable trc promoter (Brosius et al., 1985) and the strong E.coli 5S transcription terminators (Brosius et al, 1981). The trc promoter is a fusion promoter containing the consensus -35 region of the E.coli trp promoter joined to the consensus -10 region of the E.coli lacUV5 promoter (do Boer et al., 1983a). The lacUV5 sequences of this promoter contain a lac operator site, which results in transcription from this promoter being repressed in lacI^{q} strains. Transcription from this promoter in lac I^{q} strains can be stimulated by growing cells in the presence of IPTG (de Boer et al., 1983a). A restriction map and the sequence of the RBS/ cloning region of pKT52 is shown in Figure 5.

### Figure 5

### Cloning PGH cDNA into expression vector pKT52

The organization of the E.coli expression vector pKT52 is illustrated. An 800 bp PstI fragment was isolated from M13 RF containing the EcoRI insert of pPG.3. This fragment contained the entire pre-PGH coding region, minus the first two amino acids, plus 33 bp of mp19 polylinker DNA. When this fragment was inserted into the PstI site of pKT52, the full pre-PGH sequence is regenerated. The EcoRI insert of pKT52 was then isolated and cloned into M13 mp19 to facilitate mutagenesis of the pKT52/PGH cDNA junction point. The position of the trc promoter and the 5S rRNA transcription terminators (rrnT1 and rrnT2) are indicated.

Two E.coli strains were used for analysing expression levels in the work described in this Example, strains MC1061 and JM101. Expression from the trc promoter is constitutive inMC1061 cells, and repressed in the lac I^{q} strain, JM101. The repression of transcription from the trc promoter in JM101 cells was released by growing cells in the presence of 1 mM IPTG.

### Plasmid pGHX.1

A construct designed to express methionyl-PGH (m-PGH) was constructed using oligonucleotide directed mutagenesis to delete the DNA coding for amino acids 2-26 of the pre-hormone. This deletion joined the TTc codon of the first amino acid of the mature PGH molecule directly to the ATG initiator codon.

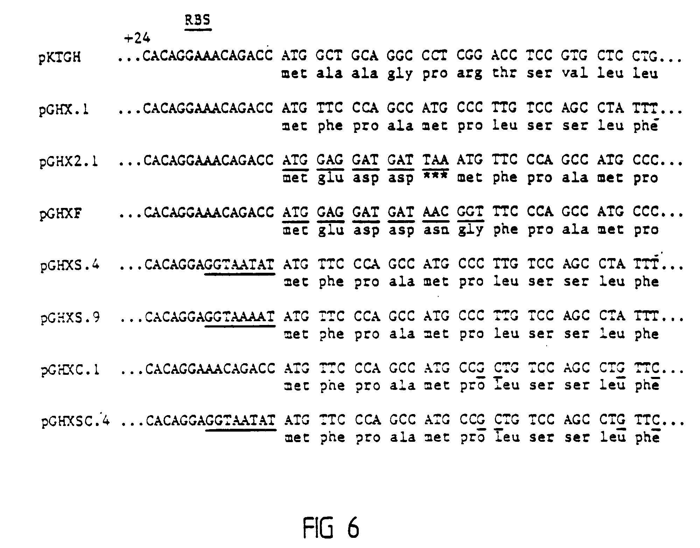

The EcoRI insert of pKTGH, which contains the entire PGH cDNA fused to the trc promoter sequences, was isolated and cloned into M13 mp19. Single-stranded DNA isolated from this 'phage was then used in mutagenesis reactions. To remove the 75 bases coding for amino acids 2-26 of the pre-hormone a 30 base long oligonucleotide, GH.30, complementary to 15 bases either side of the required deletion was used in a mutagenesis reaction. Following annealing and extension the mutagenesis reaction was transformed into JM101. The resulting plaques were then screened to detect the required deletion by plaque hybridization of duplicate lifts made from the transformation plates. Of the 150 plaques screened 35% were found to hybridize in duplicate to the GH.30 oligonucleotide probe. Single-stranded DNA was isolated from a number of positive plaques and sequenced to confirm the accuracy of the deletion using the PGH-specific oligonucleotide, GH.25, as primer. All of the positive plaques contained DNA which had the correct deletion. Replicative form (RF) DNA was isolated from this deleted 'phage (mpGHX.1) and the EcoRI insert purified and subcloned into the larger EcoRI fragment of pKTGH to create plasmid pGHX.1. The nucleotide sequence of the 5' end of this, and the other expression plasmids described are illustrated in Figure 6.

### Figure 6

### Nucleotide sequence of 5' regions of PGH expression plasmids

The nucleotide sequence of the final 24 basis of the mRNA leader and the 5' end of the coding region of each of the expression plasmids generated are illustrated. Each of the sequences were determined by priming sequencing reactions with the PGH specific oligonucleotide, GH.25. Each of the plasmids with the exception of pKTGH (pre-PGH) and pGHXF (PGH fusion protein) encode methionyl-pGH (m-PGH). Bases of the m-PGH expression plasmids which differ from the pGHX.1 sequence have been underlined.

### pGHXS (spacer) plasmids

A 38 base oligonucleotide with one redudnancy, GH.38, was designed to alter the RBS from AGGAAA to AGGAGG and the spacer from CAGACC to either TAATAT of TAAAAT. Single-stranded DNA containing the small EcoRI fragment of pGHX.1 was mutagenized and positive plaques selected and sequenced. A total of 30% of the plaques hybridized to the GH.38 probe, but of these only 20% contained either the GGTAATAT or GGTAAAAT RBS/spacer sequences. The remaining positives contained duplicate insertions which may have arisen due to the incorrect hybridization of the GH.38 oligonucleotide during the mutagenesis reaction. RF DNA was prepared from plaques containing correct versions of both the required alterations and then cloned back into pKTGH. The nucleotide sequence of the RBS/spacer region of the resulting plasmids, pGHXS.4 and pGHXS.9 are illustrated in Figure 6. Extracts prepared from both MC1061 and induced JM101 cells containing either of these two plasmids were found to contain an additional prominent band, which had a molecular weight of 22 K, the expected size of m-PGH. Laser densitometry of SDS/PAGE gels indicated that both plasmids produced identical levels of m-PGH, which ranged in the two hosts from 15 to 20% of total cellular protein.

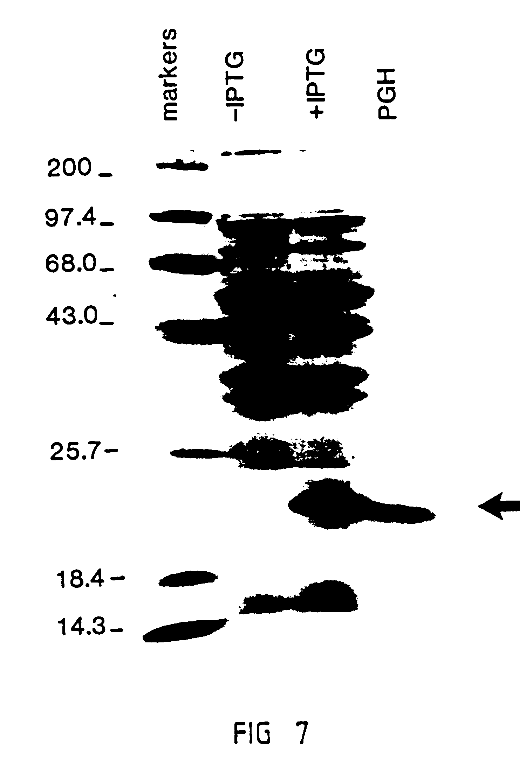

Figure 7 illustrates the level of m-PGH produced from pGHXS.4 in both uninduced and induced JM101 cells. This Figure also illustrates the similarity in molecular weight of the E.coli produced protein versus pituitary derived PGH. The m-PGH migrates at a slightly higher molecular weight than expected (approximately 200 daltons), probably due to the extremely crude nature of the protein extracts applied to the gel. This phenomenon has been previously observed in crude bacterial extracts containing human GH (Hsiung et al., 1986).

### Figure 7

### Production of m-PGH by Plasmid pGHXS.4

Protein extracts from uninduced (-IPTG) and induced (+IPTG) JM101 cells containing expression plasmid pGHXS.4 were subjected to SDS/PAGE, along with molecular weight markers and purified, pituitary derived PGH (kindly provided by R. Seamark). A prominent band of the expected molecular weight of PGH (22,000 daltons) is produced only in IPTG induced cells (3.2.2.iii).

### The construction of a human MT-IIA promoter/PGH fusion gene

The promoter chosen for these studies was the human metallothionein II-A promoter (Karin and Richards, 1982) which was kindly provided by R. Richards.

The hMT-IIA promoter was available as an 823 bp fragment with promoter sequences extending from -763 to +60 cloned in the M13 vector mp8 (A. Robins pers. comm.). The double digestion of RF 'phage containing this promoter with HindIII and EcoRI releases the promoter sequences fused to 5 bp of vector polylinker sequence. This 823 bp fragment was purified and subcloned into HindIII/EcoRI digested pUC19 to generate plasmid pUCMT.

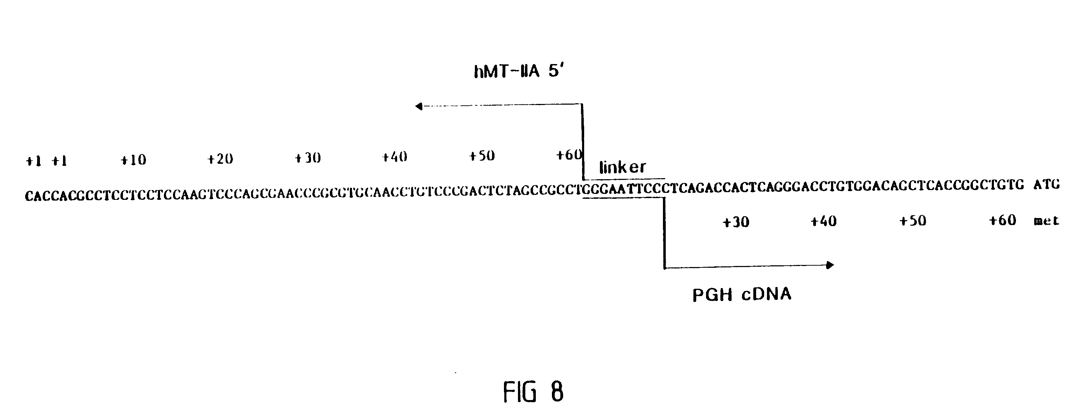

The sequences encoding PGH were isolated from the PGH cDNA clone pPG.3 as an 814 bp EcoRI fragment. This was cloned downstream of the hMT-IIA promoter, by restricting pUCMT with EcoRI, followed by ligation to the purified pPG.3 insert. Restriction analysis of plasmid DNA prepared from the resulting transformants identified a plasmid which contained the PGH cDNA inserted in the correct orientation, which was named pUCMTGH.4. The nucleotide sequence of the junction point between these two fragments was determined by the directional subcloning of a restriction fragment spanning this region into M13 mp18. The sequence data derived from this clone indicated that the expected sequence had been generated, and had contained the hMT-IIA promoter sequences and transcription start site (down to position +60) joined to the PGH 5' untranslated region (from +21 onwards) by 9 bp of polylinker/synthetic linker DNA (Figure 8).

It was decided to subclone the 1 kb SmaI/BamHI fragment from plasmid pGHB.3 (2 kb BamHI subclone of cPGH.1) which contains the fifth exon downstream of the Sma I site plus approximately 800 bp of 3' non-translated sequence from the pgh gene into the unique SmaI site of pUCMTGH.4.

The 1 kb SmaI/BamHI fragment of pGHB.3. was purified (6.3.2.ii) and the BamHI generated overhang repaired with the Klenow fragment of E.coli DNA polymerase I. This blunt-ended fragment was then subcloned into SmaI digested pUCMTGH.4. The examination of plasmid DNA isolated from a number of the resulting transformants revealed that most were equal in size to pUCMTGH.4. The transformants were therefore screened for the presence of the cPGH.1 sequences by filter hybridization using a 500 bp BamHI/PstI restriction fragment from the far 3' end of pGHB.3 as the hybridization probe. Restriction analysis of plasmid DNA prepared from the resulting positives indicated that one contained the inserted fragment in the correct orientation. This plasmid was named pHMPH.4 (HM, human metallothionein; PG, porcine growth hormone). The organization of this plasmid is illustrated in Figure 1.

### Figure 2

### Construction of eukaryotic expression plasmid pHMPG.4

A flow chart illustrating the construction of pHMPG.4 is illustrated. An approximate 800 bp HindIII/EcoRI fragment containing the hMT-IIA promoter was cloned into HindIII/EcoRi digested, dephosphorylated pUC19 to create plasmid pUCMT. This plasmid was then restricted with EcoRI, dephosphorylated, and ligated to the EcoRI insert of the PGH cDNA clone, pPG.3, to generate plasmid pUCMTGH.4. This plasmid was restricted with SmaI, dephosphorylated, and ligated to the blunt-ended 1 kb SmaI/BamHI insert of cosmid subclone pGHB.3, which contains most of the last exon of the PGH genomic gene and approximately 700 bp of PGH 3' non-coding sequence to create pHMG 4. All constructs were checked by DNA sequence analysis..

### Figure 8

### Nucleotide sequence of the hMT-IIA/PGH junction point in pUCMTGH.4

A ScaI/PstI fragment which covers the junction region between hMT-IIA and PGH cDNA sequences was isolated from pUCMTGH.4 and cloned into SmaI/PstI digested mp18 for sequence analysis. The sequence data from this clone indicated that the correct fusion had been generated, with the hMT-IIA promoter, cap site (there are two cap sites, both indicated as +1), and 5' untranslated sequences (extending down to +60), linked to the PGH cDNA sequences, which extend downstream from base +21, by 9 bp of synthetic linker sequence (4.2).

### Figure 1

### Expression vector, pHMPG.4

The restriction map and organization of expression vector pHMPG.4 is illustrated. The hMT-IIA promoter sequences are fused to a hybrid gene containing PGH cDNA sequences extending from +21 down to the unique SmaI site, joined to PGH genomic gene sequences downstream from this point. The 2.7 kb HindIII/PvuI fragment containing all of the promoter and PGH sequences, plus 120 bp of the pUC19 lacz gene joined to the 3' end, was purified from low melting temperature agarose, and used for generating transgenic animals.

### Production of transgenic pigs

The HindIII/PvuI fragment of pHMPG.4 was used to produce transgenic pigs. Single cell in vivo fertilized pig embryos were collected from superovulated large white sows. These were prepared and injected with approximately 600 copies of the pHMPG.4 insert. Approximately 30 injected embryos were surgically transferred into the oviducts of each of a number of synchronized recipient sows. Four of these sows farrowed small litters (4-5 per litter), producing a total of 17 piglets.

### Analysis of transgenic pigs

### i) Dot-blots

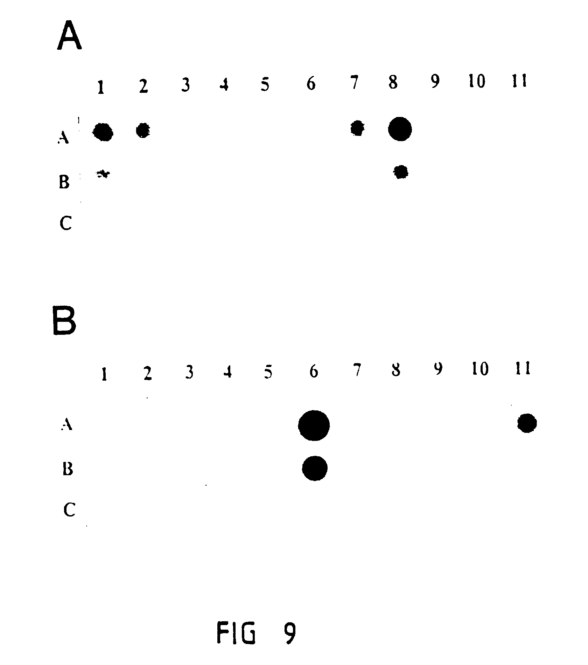

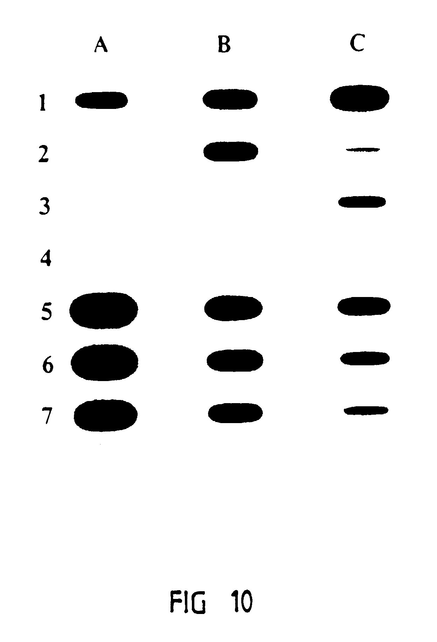

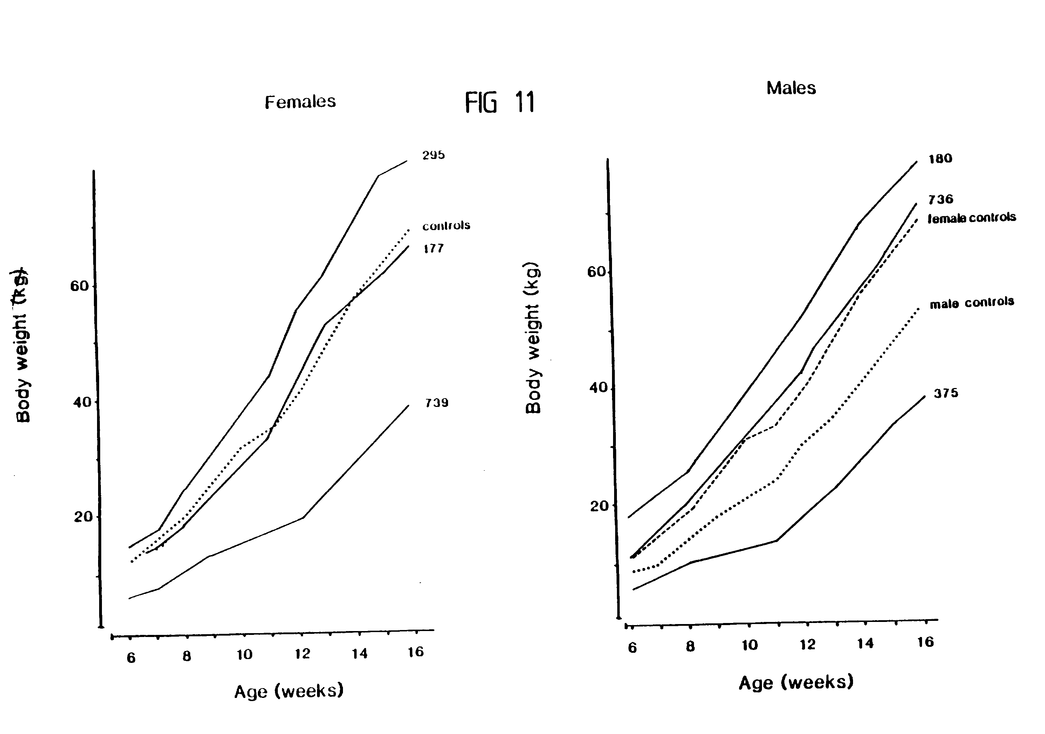

The piglets were tested for the presence of the foreign gene by dot-blot hybridization of DNA isolated from tail tissue. Both normal pig DNA and human genomic DNA were included on these dot-blots to act as negative and positive controls respectively. Following the hybridization of these dot-blots to the HindIII/AvaI fragment of the hMT-IIA promoter a number of positive signals were evident. Four of the pigs showed strong hybridization equivalent to greater than one copy per cell and a further two showed weak hybridization, slightly above background, and equivalent to less than one copy per cell (Figure 9). ii) Slot-blots

The number of copies per cell of the pHMPG.4 insert present in each of the transgenic pigs was determined by slot-blot analysis. Five µg samples of tail DNA from each of the transgenic animals was filtered onto a slot-blot along with human and pig positive and negaitve controls. A range of amounts of pHMPG.4 plasmid DNA (which also contained 5 µg of control pig genomic DNA) corresponding to genomic copy numbers equivalent to between one and forty copies per cell were also included. This slot-blot was hybridized to the nick-translated HindIII/AvaI fragment of the hMT-IIA promoter and washed at high stringency. The intensity of hybridization of the transgenic animals was compared to the plasmid standard s by laser densitometry, and found to range from approximately 0.5 copies per cell in animals #375 and #739 to 15 copies per cell in animal #295 (Table 1; Figure 9).

### iii) Southern Analysis

The organization of the foreign sequences within the transgenic pigs was studied by Southern blotting. There are no BamHI sites within the pHMPG.4 insert. The digestion of the genomic DNA of the transgenic animals with this enzyme should therefore produce bands on genomic Southerns, the length of which are governed by the distance of the nearest BamHI sites to the site of integration, the number of integration sites, and the number of integrated copies of the pHMPG.4 insert.

### Figure 9

### Analysis of potentially transgenic pigs by dot-blot

10, 5, and 1 µg samples of DNA isolated from the tails of pigs which had developed from eggs microinjected with the insert of pHMPG.4, were denatured and applied to a membrane. pHMPG.4 and human (HUM) genomic DNA positive controls and pig (PIG) genomic DNA negative controls were included on each dot-blot array. The plasmid positive controls are in row 1, blot A. In the table below the numbers in brackets refer to the number of copies per cell each plasmid dot is equivalent to. Samples in rows A4 to AII and B1 to B9 contain the test pig samples. The nick-translated hMT-IIA promoter HinIII/AvaI insert ws used as the hybridization probe. A key indicating the identity of the sample on each dot is shown below.

| A. | | | | | | | | | | | | |
|---|---|---|---|---|---|---|---|---|---|---|---|---|
| | | 1 | 2 | 3 | 4 | 5 | 6 | 7 | 8 | 9 | 10 | 11 |
| A. | 10µg | (2) | HUM | PIG | 177 | 178 | 179 | 180 | 295 | 296 | 297 | 298 |
| B. | 5µg | (1) | HUM | PIG | 177 | 178 | 179 | 180 | 295 | 296 | 297 | 298 |
| C. | 1µg | (0.5) | HUM | PIG | 177 | 178 | 179 | 180 | 295 | 296 | 297 | 298 |
| | | | | | | | | | | | | |

| B. | | | | | | | | | | | | |
|---|---|---|---|---|---|---|---|---|---|---|---|---|
| | | 1 | 2 | 3 | 4 | 5 | 6 | 7 | 8 | 9 | 10 | 11 |
| A. | 10µg | 373 | 374 | 375 | 376 | 735 | 736 | 737 | 738 | 739 | PIG | HUM |
| B. | 5µg | (1) | 374 | 375 | 376 | 735 | 736 | 737 | 738 | 739 | PIG | HUM |
| C. | 1µg | (0.5) | 374 | 375 | 376 | 735 | 736 | 737 | 738 | 739 | PIG | HUM |

### Figure 10

### Slot-blot analysis of transgenic pigs

Samples (5 µg) of transgenic pig DNA and pig (PIG) and human (HUM) negative and positive controls were denatured and applied to a membrane along with a number of samples containing various amounts of pHMPG.4 plasmid DNA combined with 5 µg of pig control DNA. The amounts of pHMPG.4 DNA applied correspond to gene copy numbers of between 1 and 40 gene copies per cell (shown in brackets below). The probe used was the nick-translated hMT-IIA promoter HindIII/AvaI insert. A key to the samples on the blot is given below. Quantitation of the intensity of hybridization of each of the samples on this membrane was performed using a laser densitometer. The results of this analysis are presented in Table below.

| | A | B | C |
|---|---|---|---|
| 1. | 177 | 180 | 295 |
| 2. | 375 | 736 | 739 |
| 3. | | PIG | HUM |
| 4. | | | |
| 5. | 500 (40) | 125 (10) | 50 (4) |
| 6. | 375 (30) | 100 (8) | 25 (2) |
| 7. | 250 (20) | 75 (6) | 12.5 (1) |

**Table 1**

| Transgenic pigs: gene copy number, growth, and serum PGH concentration | | | | |
|---|---|---|---|---|
| The number of copies (per cell) of the foreign gene present in each of the transgenic pigs estimated by slot-blot analysis, the daily weight gain (between days 50 and 120) and the serum PGH concentration of each of the transgenic pigs and their non-transgenic littermates are illustrated. The female control values are the mean of three animals, and the male control values are the mean of two animals. | | | | |

| Pig No. | sex | copy no./cell | daily weight gain (gm) | serum PGH conc. (ng/ml) |
|---|---|---|---|---|
| 177 | F | 3 | 765 | 10.4 |
| 295 | F | 15 | 953 | 27.8 |
| 739 | F | 0.5 | 686 | 6.9 |
| controls | F | - | 806 | 10.6 |
| 180 | M | 6 | 851 | 15.3 |
| 375 | M | 0.5 | 487 | 6.3 |
| 736 | M | 6 | 857 | 11.1 |
| controls | M | - | 670 | 15.3 |

### Figure 11

### Growth rate of transgenic pigs

The growth performance of each of the transgenic pigs is plotted against the growth rate of non-transgenic littermates. The dotted lines represent the average growth rate of sex-matched non-transgenic littermates, the dashed line represents the average of non-transgenic littermates of the opposite sex, and solid lines represent transgenic animals.

### Example 2

### Sp1 binding site minus hMT-IIA/PGH plasmid, pHMGPG.3

It has been reported that a consensus Spl binding site sequence, GGGCGG (Kadonaga et al., Trends Biochem. Sci. 11, 20-23, 1986) located adjacent to the basal level sequence, would generate a promoter with the desired parameters. The nucleotide sequence of an altered hMT-IIA promoter which has the Spl binding site replaced with a PstI linker, and possesses the required transcriptional characteristics, was kindly made available by M. Karin. This alteration was recreated in the hMT-IIA/ mp8 clone by oligonucleotide directed mutagenesis.

A 46 base long oligonucleotide, MT.46 was designed to replace a 15 bp region, surrounding and including the Spl binding site, with a 17 base long PstI linker sequence. Following mutagenesis of hMT-IIA/ mp8 single-stranded DNA with MT.46 and transformation into JM101 plaques containing the correct sequence substitution were selected by plaque hybridization and nucleotide sequencing. RF DNA was isolate from one of the plaques containing the correct mutation and the HindIII/EcoRI insert purified and used in a triple ligation with the EcoRI insert of pHMPG.4 and HindIII/EcoRI digested pUC19 DNA. One of the resulting plasmids from this ligation contained the correct restriction pattern, and was named pHMGPG.3 (GC sequence minus).

The insert of this plasmid is currently being introduced into transgenic mice.

It is also envisaged that the process of the present invention may be used to produce synthetic promoter constructs containing various combinations of metal-responsive elements corresponding to sequences found mouse, sheep or human metallothioneine promoters, with or without other promoter elements. For example it may be use to regulate Fe⁺⁺levels.

Finally, it is to be understood that various other modifications and/or alterations may be made without departing from the spirit of the present invention as outlined herein.

## Claims

1. A method for preparing transgenic pigs said method comprising the steps of:
(a) obtaining a recently fertilized pig ovum;
(b) isolating a first DNA sequence encoding a promotor region which promotes expression of porcine growth hormone in a pig;
(c) inserting the first DNA sequence into a plasmid cloning vector;
(d) isolating a second DNA sequence encoding a protein having porcine growth hormone activity in a transgenic pig said second DNA sequence modified at the 3' end to remove repeated sequences;
(e) inserting the second DNA sequence into the plasmid cloning vector at a suitable site;
(f) introducing the insert from plasmid cloning vector into the male pronucleus of said fertilized pig ovum prior to fusion with the female nucleus to form a single cell embryo;
(g) subsequently implanting the ovum into a female pig.

2. A method according to claim 1 wherein the first DNA sequence encodes the human metallothionein IIA gene promoter or a functional portion thereof.

3. A method according to claim 1 or 2 wherein the first DNA sequence forms an Ecori-HindIII insertion in the plasmid cloning vector and is of approximately 810-823bp in length.

4. A method according to any one of claims 1-3 wherein the first DNA seqeunce is 823bp in length.

5. A method according to any one of claims 1-4 wherein the second DNA sequence forms an EcoRI-EcoRI insertion in the plasmid cloning vector and is of approximately 800bp in length.

6. A method according to any one of claims 1-5 wherein the plasmid cloning vector is selected from pUCl9 or pKT52.

7. A method according to any preceding claim wherein the 3' end of the second DNA sequence is in the form of a Sma:Bam/Sma insertion in the second cloned sequence of approximately 1000bp in length.

8. A method according to any preceding claim wherein the repeated sequences are deleted to leave approximately 200bp of the 3' sequence.

## Patentansprüche

1. Verfahren zur Herstellung transgener Schweine mit den Schritten in denen man:
a) eine kurz zuvor befruchtete Schweineeizelle erhält,
b) eine erste DNA-Sequenz isoliert, die einen Promotorbereich kodiert, die die Expression von Schweinewachstumshormon in einem Schwein fördert,
c) die erste DNA-Sequenz in einen Plasmidklonierungsvektor einfügt,
d) eine zweite DNA-Sequenz isoliert, die ein Protein mit Schweinewachstumshormonaktivität in einem transgenen Schwein kodiert, wobei diese zweite DNA-Sequenz an 3'-Ende verändert ist, um sich wiederholende Sequenzen zu entfernen,
e) die zweite DNA-Sequenz an einer geeigneten Stelle in den Plasmidklonierungsvektor einfügt,
f) die Einfügung aus dem Plasmidklonierungsvektor in den männlichen Vorkern der befruchteten Schweineeizelle einführt, bevor er sich mit dem weiblichen Kern vereinigt, um einen einzelligen Embryo zu bilden,
g) schließlich die Eizelle in ein weibliches Schwein implantiert.

2. Verfahren nach Anspruch 1, **dadurch gekennzeichnet**, daß die erste DNA-Sequenz den menschlichen Metallothionein IIA-Genpromotor oder einen funktionellen Bereich davon kodiert.

3. Verfahren nach Anspruch 1 oder 2, **dadurch gekennzeichnet, daß** die erste DNA-Sequenz in dem Plasmidklonierungsvektor eine EcoRI-HindIII-Insertion bildet und etwa 810-8238p lang ist.

4. Verfahren nach einem der Ansprüche 1 bis 3, **dadurch gekennzeichnet**, daß die erste DNA-Sequenz 823Bp lang ist.

5. Verfahren nach einem der Ansprüche 1 bis 4, **dadurch gekennzeichnet**, daß die zweite DNA-Sequenz in dem Plasmidklonierungsvektor eine EcoRI-EcoRI-Insertion bildet und etwa 800Bp lang ist.

6. Verfahren nach einem der Ansprüche 1 bis 5, **dadurch gekennzeichnet**, daß der Plasmidklonierungsvektor pUC19 oder pKT52 ist.

7. Verfahren nach einem der Ansprüche 1 bis 6, **dadurch gekennzeichnet**, daß das 3'-Ende der zweiten DNA-Sequenz in Form einer Sma:Bam/Sma-Insertion in der zweiten klonierten Sequenz mit einer Länge von etwa 1000Bp vorliegt.

8. Verfahren nach einem der Ansprüche 1 bis 7, **dadurch gekennzeichnet**, daß die sich wiederholenden Sequenzen entfernt sind, so daß etwa 200Bp der 3'-Sequenz zurückbleiben.

## Revendications

1. Procédé de préparation de porcs transgéniques ledit procédé comprenant les étapes consistant à:
(a) obtenir un ovule de truie récemment fertilisé;
(b) isoler une première séquence d'ADN codant une région promoteur qui facilite l'expression de l'hormone de croissance porcine chez le porc;
(c) insérer la première séquence d'ADN dans un vecteur de clonage plasmidique;
(d) isoler une seconde séquence d'ADN codant une protéine ayant l'activité de l'hormone de croissance porcine chez un porc transgénique ladite seconde séquence d'ADN étant modifiée à l'extrémité 3' afin de retirer les séquences répétées;
(e) insérer la seconde séquence d'ADN dans le vecteur de clonage plasmidique à un site approprié;
(f) introduire l'insert à partir du vecteur de clonage plasmidique dans le pronucleus mâle dudit ovule de truie fertilisé avant de fusionner avec le pronucleus femelle pour former un embryon cellulaire unique;
(g) implanter ensuite l'ovule dans une truie.

2. Procédé selon la revendication 1 dans lequel la première séquence d'ADN code le promoteur du gène de la métallothionéine IIA humaine ou une partie fonctionnelle de celui-ci.

3. Procédé selon la revendication 1 ou 2 dans lequel la première séquence d'ADN forme une insertion EcoRI - HindIII dans le vecteur de clonage plasmidique et a une longueur approximative comprise entre 810 et 823 paires de bases.

4. Procédé selon l'une quelconque des revendications 1 à 3 dans lequel la première séquence d'ADN a une longueur de 823 paires de bases.

5. Procédé selon l'une quelconque des revendications 1 à 4 dans lequel la seconde séquence d'ADN forme une insertion EcoRI - EcoRI dans le vecteur de clonage plasmidique et a une longueur approximative de 800 paires de bases.

6. Procédé selon l'une quelconque des revendications 1 à 5 dans lequel le vecteur de clonage plasmidique est choisi entre pUC19 ou pKT52.

7. Procédé selon l'une quelconque des revendications précédentes dans lequel l'extrémité 3' de la seconde séquence d'ADN a la forme d'une insertion Sma:Bam/Sma dans la seconde séquence clonée de longueur approximative de 1000 paires de bases.

8. Procédé selon l'une quelconque des revendications précédentes dans lequel les séquences répétées sont supprimées pour ne laisser de la séquence 3' que 200 paires de bases environ.
